# EUROPÄISCHE PATENTSCHRIFT

(11) **EP 0 457 730 B1**
(45) Veröffentlichungstag und Bekanntmachung des Hinweises auf die Patenterteilung: **09.11.1994**
(21) Anmeldenummer: 91810358.1
(22) Anmeldetag: 08.05.1991
(51) Int. Cl.: C07D 251/46, C07C 215/76

(54) **Verfahren zur Herstellung von Alkylydroxyanilino-triazinthioderivaten**
Process for preparation of thioderivatives of alkylhydroxyanilinotriazine
Procédé pour la préparation des dérivés thio de l'alkylhydroxyanilino-triazine

(30) Priorität: 18.05.1990 CH 1701/90
(43) Veröffentlichungstag der Anmeldung: 21.11.1991
(73) Patentinhaber: CIBA-GEIGY AG, 4002 Basel (CH)
(72) Erfinder: Tritschler, Wolfgang, Dr., W-7852 Binzen (DE); Steiner, Heinz, CH-4142 Münchenstein (CH); Prestel, Helmut, Dr., W-7520 Bruchsal (DE); Maul, Rudolf, Dr., W-6143 Lorsch/Hessen (DE)

(56) Entgegenhaltungen:
- WO-A-89/10347
- DE-A- 2 202 219
- US-A- 3 255 191
- JOURNAL OF THE AMERICAN CHEMICAL SOCIETY Bd. 76, Nr. 19, 5. Oktober 1954, COLUMBUS OHIO Seiten 4985 - 4988; HARRY E. ALBERT: 'Some new Amino Alkylphenols'
- THE JOURNAL OF ORGANIC CHEMISTRY Bd. 19, Nr. 7, Juli 1954, BALTIMORE USA Seiten 1067 - 1079; HENRY GILMAN ET AL.: 'The Synthesis of N-methyl-3-isopropyl-4-dimethylaminophenyl carbamate and some related derivatives'

## Beschreibung

Die vorliegende Erfindung betrifft ein Verfahren zur Herstellung von Alkylhydroxyanilino-triazin-thioderivaten in hohen Ausbeuten ohne Isolierung der entstehenden Zwischenprodukte.

Die Alkylhydroxyanilino-triazin-thioderivate werden ausgehend von den entsprechenden Phenolen, durch Nitrosierung zu den Alkyl-nitrosophenolen und deren Hydrierung zu den Aminophenolen sowie einer anschliessenden Kondensation mit Cyanurchlorid und Alkyl- oder Phenylmercaptanen erhalten.

In den US-A 3,156,690, US-A 3,257,354, US-A 3,255,191 und US-A 3,240,749 wird die Herstellung von Alkylhydroxyanilino-triazin-thio-derivaten beschrieben. Dabei werden jeweils alle Zwischenprodukte isoliert und die Reduktion des Nitrosophenols zum Anilin erfolgt mit Natriumhydrogensulfit Unabhängig davon sind in der Literatur weitere Reduktionsverfahren beschrieben, die von substituierten Nitrosophenolen zu den entsprechenden Aminophenolen führen. So beschreibt E. Albert im J. Am. Chem.Soc. 76, 4985 (1954) die Möglichkeit, Nitrosophenole mittels Natriumhydrogensulfit oder durch Zink in Essigsäure zu reduzieren. Katalytische Hydrierungen von Nitrosophenolen zu Aminophenolen mit Raney-Nickel führen H. Gilman et al (J.Org. Chem. 19, 1067 (1954)) durch. Auch in der FR-A-2,122,637 wird eine katalytische Hydrierung mit Raney-Nickel beschrieben und die Möglichkeit der Verwendung anderer Katalysatoren wie z.B. Palladium, Platin oder Kobalt erwähnt. In der EP-A-0 191 983 wird die Herstellung von Dithiodialkanoamidophenol-Verbindungen über die Zwischenstufen der entsprechenden Nitroso- und Aminophenole beschrieben. Der Hydrierungsschritt wird katalytisch an Palladium/Kohle Katalysatoren durchgeführt. Aus der WO-A 89/10347 ist ferner ein Verfahren zur Herstellung von N-acylierten 3,5-Dialkyl-4-aminophenolen durch Hydrierung der entsprechenden Nitrosophenole und N-Acylierung der erhaltenen Aminophenole im gleichen Lösungmittel bekannt.

Die Alkylhydroxyanilino-triazin-thio-derivate, insbesondere 6-(3',5'-Dialkyl-4'-hydroxyphenylamino)-2,4-dialkylthio-1,3,5-triazine, sind wertvolle, zum Teil im Handel erhältliche Substanzen zum Stabilisieren von Materialien, die gegen oxidativen, thermischen und/oder lichtinduzierten Abbau empfindlich sind. Es besteht daher ein Interesse, diese Verbindungen auf einem grosstechnisch günstigen Weg mit möglichst geringer Oekologiebelastung herzustellen.

Es wurde nun gefunden, dass sich das Verfahren besonders vorteilhaft durchführen lässt, wenn man ohne Isolierung der Zwischenprodukte arbeitet und den Reduktionsschritt mittels Wasserstoff an Palladium-Katalysatoren durchführt.

Gegenstand der vorliegenden Erfindung ist ein Verfahren zur Herstellung von Alkylhydroxyanilino-triazin-thio-derivaten der Formel I 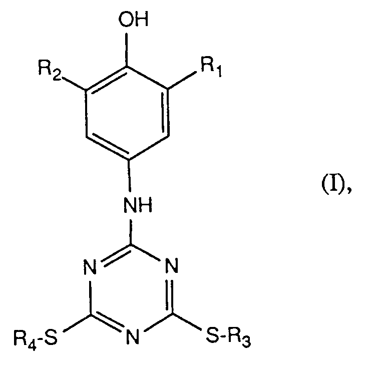
worin R₁ und R₂ unabhängig voneinander Wasserstoff, C₁-C₁₂-Alkyl, C₅-C₆-Cycloalkyl oder Phenyl bedeuten und
R₃ und R₄ unabhängig voneinander für C₁-C₁₈-Alkyl, C₅-C₆-Cycloalkyl, Phenyl, mit C₁-C₆ Alkyl und/oder Hydroxy substituiertes Phenyl oder Phenyl-C₁-C₄-Alkyl stehen, dadurch gekennzeichnet, dass man eine Verbindung der Formel III 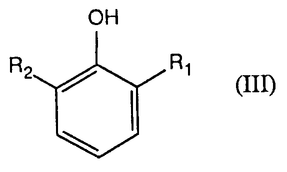
mit einem Nitrit in wässrigem oder wässrig/organischem Medium zu einer Verbindung der

### Formel II

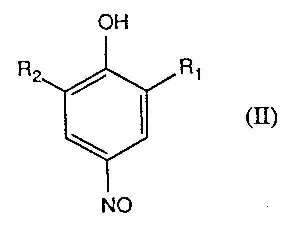

umsetzt, letztere mit einem Lösungsmittel aus der Reaktionslösung extrahiert, sie ohne Isolierung im genannten Lösungmittel in Gegenwart eines Palladium-Katalysators katalytisch zum entsprechenden p-Aminophenol hydriert und letzteres ohne dessen Isolierung mit Cyanurchlorid und einer Verbindung der Formel HSR₃ oder HSR₄ oder einer Mischung von Verbindungen der Formeln HSR₃ und HSR₄ umsetzt.

R₁ und R₂ als C₁-C₁₂-Alkyl können linear oder verzweigt sein und beispielsweise Methyl, Ethyl, n-Propyl, Isopropyl, n-Butyl, Isobutyl, tert-Butyl, n-Pentyl, Isopentyl, tert-Pentyl, Hexyl, Heptyl, n-Octyl, Isooctyl, Nonyl, Decyl, Undecyl oder Dodecyl, insbesondere tert-Butyl bedeuten.

R₁ und R₂ als C₅-C₆-Cycloalkyl sind Cyclopentyl oder Cyclohexyl.

R₁ und R₂ stehen bevorzugt für Wasserstoff oder C₁-C₁₂-Alkyl, insbesondere für C₁-C₄-Alkyl.
In einer anderen bevorzugten Ausführungsform sind R₁ und R₂ gleich. Ganz besonders bevorzugt sind R₁ und R₂ gleich und bedeuten tert-Butyl.

Sind R₃ und R₄ C₁-C₁₈-Alkyl so können sie linear oder verzweigt sein und z.B. die für R₁ und R₂ angegebenen Bedeutungen haben und zusätzlich beispielsweise Tridecyl, Tetradecyl, Pentadecyl, Hexadecyl, Heptadecyl oder Octadecyl, bevorzugt C₁-C₁₂-Alkyl, insbesondere n-Octyl bedeuten.

R₃ und R₄ als C₅-C₆-Cycloalkyl haben die gleichen Bedeutungen wie für R₁ und R₂ beschrieben.

R₃ und R₄ als ein- oder mehrfach mit C₁-C₆-Alkyl substituiertes Phenyl können beispielsweise für mit 1 bis 3, insbesondere 1 oder 2 Alkylgruppen, vor allem mit Methylgruppen substituiertes Phenyl stehen. Beispiele dafür sind Tolyl, Xylyl oder Mesityl. R₃ und R₄ als mit C₁-C₆-Alkyl substituiertes Phenyl können auch beispielsweise für Ethylphenyl, Diethylphenyl, Propylphenyl, Dipropylphenyl, Butylphenyl, Ethylmethylphenyl, Methylpropylphenyl, Butylmethylphenyl oder Ethylpropylphenyl stehen.

R₃ und R₄ als ein- oder mehrfach mit Hydroxy oder C₁-C₆-Alkyl und Hydroxy substituiertes Phenyl können beispielsweise Hydroxyphenyl, Dihydroxyphenyl, Resorcyl, Kresyl, Ethylhydroxyphenyl, Propylhydroxyphenyl, Butylhydroxyphenyl, Pentylhydroxyphenyl, Hexylhydroxyphenyl, Dimethylhydroxyphenyl, Diethylhydroxyphenyl, Dipropylhydroxyphenyl, Ethylmethylhydroxyphenyl, Methylpropylhydroxyphenyl, Butylmethylhydroxyphenyl, Methylpentylhydroxyphenyl, Ethylpropylhydroxyphenyl oder Butylethylhydroxyphenyl bedeuten.

Stehen R₃ und R₄ für Phenyl-C₁-C₄-Alkyl, so können sie zum Beispiel Benzyl, Phenylethyl, α-Methylbenzyl, Phenylpropyl, α,α-Dimethylbenzyl, Phenyl(methylpropyl) oder Phenylbutyl, insbesondere Benzyl, sein. Bevorzugt sind R₃ und R₄ C₁-C₁₂-Alkyl.

Bevorzugt ist ein Verfahren, worin R₁ und R₂ unabhängig voneinander Wasserstoff oder C₁-C₁₂-Alkyl bedeuten und R₃ und R₄ unabhängig voneinander für C₁-C₁₂-Alkyl stehen.

In einer weiteren bevorzugten Ausführungsform sind R₃ und R₄ gleich.

Zur Durchführung der Nitrosierung geeignete Nitrite sind z.B. Alkali- oder Erdalkalinitrite wie z.B. NaNO₂, KNO₂, Ba(NO₂)₂ oder Ca(NO₂)₂, bevorzugt sind Alkalinitrite, insbesondere Natriumnitrit.

Bevorzugt wird das erfindungsgemässe Verfahren in einem Lösungsmittel durchgeführt, das unter den Bedingungen der Hydrierstufe inert ist.

Geeignete Lösungsmittel dafür sind z.B. Ketone, aromatische, cycloaliphatische oder aliphatische Kohlenwasserstoffe, Ether oder Ester, Mischungen solcher Lösungsmittel, sowie Gemische dieser Lösungsmittel mit Wasser. Von den Ketonen sind besonders aliphatische und cycloatiphatische Ketone zu nennen.

Aliphatische Ketone sind beispielsweise Aceton, Diethylketon, Methylethylketon, Methylisoburylketon oder Hexanon, bevorzugt sind Methylethylketon und Methylisobutylketon. Ein Beispiel für cycloaliphatische Ketone ist Cyclohexanon. Als aromatische Kohlenwasserstoffe kommen beispielsweise Benzol, Toluol oder Xylol in Frage.

Als Ether kommen z.B. Diethylether oder tert-Butylmethylether in Frage. Ein geeigneter Ester ist beispielsweise Essigsäureethylester. Aliphatische oder cycloaliphatische Kohlenwasserstoffe sind z.B. Hexan, Heptan oder Cyclohexan.

Besonders bevorzugt sind Ketone als Lösungsmittel. Ueberraschenderweise verhalten sich Ketone unter den Hydrierbedingungen völlig inert, d.h. es wird weder eine Hydrierung des Ketons, noch eine reduktive Alkylierung des Aminophenol-Derivates mit dem Keton beobachtet.

Besonders bevorzugt ist ein Verfahren, worin als Lösungsmittel Methylisobutylketon, Methylethylketon oder Gemische dieser Lösungsmittel mit Wasser verwendet werden.

Die katalytische Hydrierung des Verfahrens wird zweckmässig bei Temperaturen von 20-100°C, vorzugsweise 20-80°C, insbesondere 30-70°C durchgeführt. Die Wasserstoffdrucke, bei denen die katalytische Hydrierung durchgeführt wird, sind nicht kritisch und können in weiten Grenzen schwanken, liegen aber zweckmässig bei 1-200 bar, vorzugsweise bei 1-100 bar, insbesondere bei 1-50 bar.

Welche Wasserstoffdrucke angewendet werden, hängt auch von der zur Verfügung stehenden Hydrieranlage ab.

Die Hydrierzeit kann in weiten Grenzen schwanken, sie ist abhängig vom verwendeten Katalysator, vom Wasserstoffdruck, von der Reaktionstemperatur und der verwendeten Anlage. Sie kann z.B. von 1 Minute bis 2 Stunden betragen, insbesondere 5 Minuten bis 1 Stunde, z.B. 10 Minuten bis 30 Minuten. Selbstverständlich wird durch Erhöhung des Wasserstoffdruckes oder/und der Temperatur die Reaktionszeit verkürzt.

Bevorzugt ist ein Verfahren, worin die Katalysatormenge 0,01-5,0 Gew.-%, vorzugsweise 0,02-2 Gew.-%, insbesondere 0,1-2,0 Gew.-%, z.B. 0,2-1,5 Gew.-%, vor allem 0,2-1 Gew.-%, bezogen auf die Verbindung der Formel II, beträgt.

Beim erfindungsgemässen Verfahren wird die katalytische Hydrierung in Gegenwart eines Palladium-Katalysators durchgeführt. Dieser wird zweckmässig in einer für diesen Verwendungszweck üblichen Formen eingesetzt, d.h. das jeweilige Metall ist auf einem Täger aufgebracht, z.B. auf Aktivkohle, Kieselgur, Aluminiumoxid, Bariumsulfat usw. Die Katalysatoren können durch weitere Metalle aktiviert werden, z.B. durch Mg, Zr oder Mo. Bevorzugt werden im erfindungsgemässen Verfahren Palladium-Katalysatoren auf einem Träger, insbesondere Palladium/Kohle-Katalysatoren eingesetzt (Pd/C).

Die Katalysatormenge auf dem Träger (Belegung) beträgt zweckmässig 0,5-10 %, insbesondere z.B. 0,5-5,0 %, ganz besonders 0,5-2,0 %.

Das erfindungsgemässe Verfahren wird zweckmässig so ausgeführt, dass zunächst das Phenol der Formel III mit dem Nitrosierungsreagenz (insbesondere NaNO₂) in wässrigem oder wässrig/organischem Reaktionsmedium zweckmässig in Gegenwart einer Säure umgesetzt wird. Dazu wird z.B. das Phenol im Lösungsmittel bzw. Lösungsmittelgemisch vorgelegt und das Nitrosierungsreagenz, in Wasser gelöst, zugetropft. Nach Abdestillieren eines Teils des Lösungsmittels (z.B. wird bei Einsatz eines wässrig/organischen Lösungsmittelgemisches das organische Lösungsmittel als Azeotrop mit Wasser abdestilliert) wird das entstandene p-Nitrosozwischenprodukt der Formel II mit einem Lösungsmittel, welches unter der gewählten Hydrierungsbedingungen inert ist, aus dem verbleibenden wässrigen Reaktionsgemisch extrahiert. Die extrahierte Lösungsmittelphase kann dann, vorzugsweise unter Inertbedingungen, in einen Autoklaven, der den Katalysator enthält, überführt werden. Durch Aufdrücken von Wasserstoff, Temperaturerhöhung und Rühren wird die p-Nitrosoverbindung der Formel II zum entsprechenden p-Aminophenol hydriert. Nach beendeter Reaktion wird der Katalysator abfiltriert und das Filtrat mit einem Alkyl- bzw. Phenylmercaptan der Formel HSR₃ oder/und HSR₄ und Cyanurchlorid umgesetzt. Das geschieht unter üblichen Bedingungen zweckmässig so, dass man das Cyanurchlorid im gleichen Lösungsmittel vorlegt, in welchem die Nitrosoverbindung extrahiert und hydriert wurde, und das p-Aminophenol enthaltende Filtrat sowie das Alkyl- bzw. Phenylmercaptan unter Rühren und Kühlen zutropft. Die Isolation des Reaktionsproduktes erfolgt durch übliche Methoden wie Destillation und/oder Kristallisation.

Die Ausführung des Hydrierungsschrittes kann sowohl im Batch- als auch in einem halbkontinuierlichen Vefahren erfolgen. Beim halbkontinuierlichen Verfahren wird die Extraktionslösung nach der Nitrosierungsstufe kontinuierlich im Verlauf der Hydrierung gegen den Wasserstoffdruck in den Hydrierreaktor, der den Katalysator enthält, dosiert.

Wie weiter oben erwähnt, können die Hydrierung und die Umsetzung mit Cyanurchlorid und dem Mercaptan auch in Mischungen der genannten Lösungsmittel mit Wasser durchgeführt werden. Vom Extraktionsschritt her enthält das Reaktionsgemisch ohnehin eine gewisse Menge Wasser. Es kann aber vor der Hydrierung oder/und vor der letzten Stufe noch zusätzliches Wasser zugegeben werden. Zweckmässig gibt man, wenn überhaupt, so viel Wasser zu, dass am Ende des Verfahrens das Lösungsmittel/Wasser-Gemisch azeotrop abdestilliert werden kann.

Es ist für den Fachmann selbstverständlich, dass man in der letzten Stufe den stöchiometrischen Gegebenheiten nach zweckmässig mindestens 2 Mol an Verbindung HSR₃ oder/und HSR₄ pro Mol des p-Aminophenols und des Cyanurchlorids einsetzt. Werden Verbindungen der Formel I gewünscht, in denen R₃≠R₄, so führt man die Umsetzung zweckmässig mit einer Mischung aus 2 verschiedenen Mercaptanen durch. Bevorzugt ist jedoch R₃=R₄. In diesem Fall setzt man etwa 2 Moläquivalent eines Mercaptans ein.

Die Reaktionsbedingungen im Nitrosierungsschritt und in der abschliessenden Umsetzung mit Cyanurchlorid und Mercaptan sind nicht kritisch. Beide Umsetzungen werden in an sich bekannter Weise ausgeführt, wie sie z.B. in den US-A 3,156,690, 3,257,354, 3,255,191 und 3,240,749 beschrieben sind, sofern die erfindungswesentlichen Massnahmen beachtet werden.

Zweckmässig führt man die Nitrosierung bei Temperaturen von -20 bis +50°C, vorzugsweise -10 bis +30°C durch. Wie bereits erwähnt, läuft die Reaktion vorteilhaft in wässrig/organischem Medium ab, wobei als organische Lösungsmittelkomponente ein mit Wasser mischbares Lösungsmittel mitverwendet werden kann, z.B. ein Alkohol wie ein C₁-C₅-Alkanol, insbesondere Methanol, Ethanol, n-Propanol, Isopropanol, n-Butanol, i-Butanol usw., oder ein aliphatisches Keton, insbesondere Aceton, Diethylketon, Methylethylketon oder Methylisobutylketon, vor allem Aceton. Die Nitrosierung wird üblicherweise in Gegenwart einer Säure, z.B. einer Mineralsäure wie Schwefelsäure, Phosphorsäure oder insbesondere Salzsäure durchgeführt. Die Extraktion der Nitrosoverbindung der Formel II kann unmittelbar aus der Reaktionsmischung erfolgen, manchmal erweist es sich jedoch als zweckmässig, einen Teil des Reaktionsmediums abzudestillieren, z.B. um ein mitverwendetes Lösungsmittel zu entfernen (etwa azeotrope Destillation eines Alkohol/Wassergemisches). Dann wird der Rückstand extrahiert, gegebenenfalls nach Zugabe von Wasser.

In der letzten Stufe wird beispielsweise bei Temperaturen von -20 bis +70°C, insbesondere -10 bis +50°C, z.B. - 10 bis +30°C gearbeitet. Enthält das Reaktionsgemisch noch kein Wasser, ist es oft zweckmässig, solches zuzufügen, beispielsweise zusammen mit dem Cyanurchlorid. Nach Beendigung der Reaktion kann dann das Lösungsmittel als azeotropes Gemisch mit Wasser gut durch Destillation entfernt werden. Da bei der Reaktion HCl entsteht, ist es besonders zweckmässig, der Reaktionsmischung eine Base zuzusetzen. Als solche kann z.B. ein Alkali- oder Erdalkalihydroxid, z.B. NaOH, KOH, Ca(OH₂), Mg(OH)₂ usw., oder ein organisches Amin, insbesondere ein Tri-C₁-C₆-Alkylamin (z.B. Trimethyl-, -ethyl-, -propyl- oder -butylamin) fungieren. Bevorzugt verwendet man ein Alkalihydroxid, insbesondere KOH, vor allem NaOH. Vor der Isolierung des Produktes ist es zweckmässig, die Base mit einer Säure zu neutralisieren. Prinzipiell kann die Zugabe des Cyanurchlorids, des Mercaptans und der Base zur Lösung des p-Aminophenols gleichzeitig oder in beliebiger Reihenfolge geschehen. Es ist jedoch zweckmässig, zunächst das Mercaptan zum p-Aminophenol zu geben, anschliessend diese Mischung (gelöst in einem wässrig/organischen oder organischen Lösungsmittel) zum Cyanurchlorid zuzutropfen und zuletzt die Base zuzusetzen.

Bisher wird bei der Herstellung von Alkyl-hydroxyanilino-triazin-thio-derivaten der Reduktionsschritt mit einem anorganischen Reduktionsmittel (z.B. Natriumhydrogensulfit) durchgeführt. Dies bringt eine starke Oekologiebelastung durch das Anfallen von Salzlasten mit sich. Die katalytische Hydrierung bietet hier ein wirtschaftlich und ökologisch günstigeres Verfahren. Als entscheidender Vorteil des erfindungsgemässen Verfahrens ist die Durchführung desselben ohne Isolierung der Zwischenprodukte, vor allen Dingen ohne Isolierung der p-Nitrosozwischenverbindung zu nennen.

Für die Durchführung des erfindungsgemässen Verfahrens soll das gewählte Lösungsmittel für alle Zwischenprodukte auch eine ausreichende Löslichkeit aufweisen. Gleichzeitig muss sich das gewählte Lösungsmittel unter den gewählten Hydrierparametern inert verhalten und soll bei der darauffolgenden Kondensation nicht verändert werden. Es soll also a) imstande sein, die p-Nitrosoverbindung zu extrahieren, b) unter den Reaktionsbedingungen der Hydrierung inert sein und c) als Reaktionsmedium für die Umsetzung des Hydrierungsproduktes mit Alkyl- bzw. Phenylmercaptanen und Cyanurchlorid geeignet sein.

Ueberraschenderweise kann nun im erfindungsgemässen Verfahren durch die beschriebenen Massnahmen das bisherige Verfahren zur Herstellung von Alkyl-hydroxyanilino-triazin-thio-derivaten, in welchem die Zwischenprodukte isoliert wurden, ohne Isolierung der Zwischenprodukte mit hohen Ausbeuten durchgeführt werden.

Die nachfolgenden Beispiele erläutern die Erfindung weiter. Angaben in Teilen oder Prozenten beziehen sich, ebenso wie in der übrigen Beschreibung und in den Patentansprüchen, auf das Gewicht, sofern nichts anderes angegeben ist.

### Beispiel 1: 6-(4'-Hydroxy-3',5'-di-tert.butylanilino)-2,4-bis-(n-octylthio)-1,3,5-triazin

In einem 1 l Glasreaktor werden 44,4 g 2,6-Di-tert.-butylphenol in einer Mischung aus 125 g Isopropanol und 20 g Wasser suspendiert, auf 0°C abgekühlt, mit 27,7 g Salzsäure (32 %) und 75 g Wasser versetzt und durch Zutropfen von 38,8 g Natriumnitritlösung (40 %) bei -5 bis +20°C nitrosiert.

Nach beendeter Nitrosierung werden 200 g Wasser zugegeben und das Isopropanol als Azeotrop mit Wasser abdestilliert. Zur Ueberführung des Nitroso-Zwischenproduktes aus der wässrigen in die organische Phase werden 180 g Methylethylketon zugegeben. Es resultiert bei 60-65°C eine orangegelbe Oberphase und eine klare, wässrige Unterphase, die abgetrennt und verworfen wird.

Die Oberphase wird in einen 300 ml Autoklaven, in dem sich 0,5 g eines 1 % Pd auf Aktivkohle-Katalysators befinden, unter inerten Bedingungen (N₂-Spülung) transferiert. Nach Aufdrücken von 10 bar Wasserstoff wird bei 50°C unter intensiver Rührung hydriert Das Ende der Hydrierung ist durch den Stillstand der Wasserstoffaufnähme nach 2 Moläquivalenten bezüglich der Nitroso-Zwischenverbindung leicht zu erkennen. Die Hydrierzeit beträgt ca. 0,3 Stunden.

Anschliessend wird der Katalysator abfiltriert und das Filtrat mit 62,3 g Octanthiol versetzt. Diese Lösung wird bei -5 bis 0°C zu einer Mischung aus 97 g Methylethylketon, 150 g Eis sowie 38,7 g Cyanurchlorid während ca. 30 Minuten zugetropft. Nach Zugabe von 88,7 g Natriumhydroxydlösung (30 %) wird 1 Stunde auf Rückfluss erhitzt, danach das Methylethylketon als Azeotrop mit Wasser abdestilliert. Die verbleibende Reaktionsmasse wird mit Essigsäure angesäuert, die wässrige Unterphase bei 95-97°C abgetrennt, die Produktschmelze durch Destillation weiter entwässert und mit 225 g Isopropanol versetzt. Nach Abkühlen auf 0°C (Kristallisationsbeginn bei ca. 60°C), werden die Kristalle abfiltriert, mit 240 g Isopropanol nachgewaschen sowie getrocknet. Ausbeute 92 % der Theorie.

Die Beispiele 2-7 werden analog zu Beispiel 1 durchgeführt, wobei die Parameter in der Hydrierstufe variiert werden. Diese sind in Tabelle 1 angegeben.

**Tabelle 1**

| Beispiel | % Pd auf Kohle | Katalysatormenge [g] | Temperatur [°C] | Druck [bar] | Hydrierzeit [h] | Ausbeute [%] |
|---|---|---|---|---|---|---|
| 2 | 1 | 0,5 | 40 | 10 | 0,7 | 92 |
| 3 | 1 | 0,5 | 50 | 5 | 0,6 | 92 |
| 4 | 1 | 0,5 | 50 | 40 | 0,2 | 92 |
| 5 | 3 | 0,5 | 50 | 10 | 0,3 | 89 |
| 6 | 1 | 0,2 | 50 | 10 | 1,0 | 92 |
| 7 | 1 | 1,0 | 50 | 10 | 0,2 | 92 |

Beispiel 8: Beispiel 1 wird wiederholt, wobei jedoch anstelle des Methylethylketons bei der Extraktion der Nitroso-Zwischenverbindung aus der wässrigen in die organische Phase Methylisobutylketon eingesetzt wird. Bei der Hydrierung und anschliessenden Aufarbeitung der Reaktionsmasse ist deshalb ebenfalls Methylisobutylketon anstelle des Methylethylketons eingesetzt. Bei der Aufarbeitung der Reaktionsmasse (Rückfluss bzw. Abdestillation des Methylisobutylketons vor der Kristallisation des Produktes) wird die Temperatur entsprechend dem Siedeverhalten des geänderten Lösungsmittels angepasst. Die Ausbeute beträgt 91 % der Theorie.

Beispiel 9: Beispiel 1 wird wiederholt, wobei jedoch anstelle der Batchhydrierung ein halbkontinuierliches Verfahren angewendet wird. Dazu wird nach der Extraktion der Nitroso-Zwischenverbindung aus der wässrigen in die organische Phase die resultierende Lösung nicht auf einmal in den Hydrierreaktor transferiert, sondern kontinuierlich im Verlauf der Hydrierung gegen den Wasserstoffdruck in den Reaktor dosiert, in dem sich der gesamte Katalysator sowie eine zum Dispergieren des Katalysators nötige Methylethylketon-Menge befindet.

Dabei findet die Reduktion der Nitroso-Zwischenverbindung zur Amin-Zwischenverbindung statt. Nach Ende der Wasserstoffaufnahme werden die weiteren Schritte analog Beispiel 1 durchgeführt.

Das Produkt wird in einer Ausbeute von 89 % der Theorie isoliert.

## Patentansprüche

1. Verfahren zur Herstellung von Alkylhydroxyanilino-triazin-thio-derivaten der Formel I 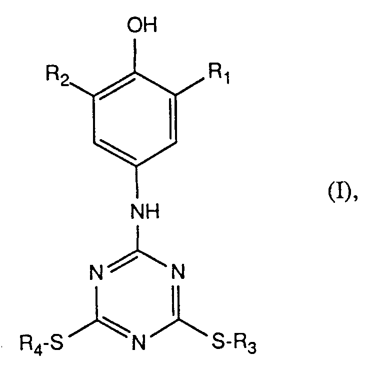 worin R₁ und R₂ unabhängig voneinander Wasserstoff, C₁-C₁₂-Alkyl, C₅-C₆-Cycloalkyl oder Phenyl bedeuten und
R₃ und R₄ unabhängig voneinander für C₁-C₁₈-Alkyl, C₅-C₆-Cycloalkyl, Phenyl, mit C₁-C₆ Alkyl und/oder Hydroxy substituiertes Phenyl oder Phenyl-C₁-C₄-Alkyl stehen, dadurch gekennzeichnet, dass man eine Verbindung der Formel III 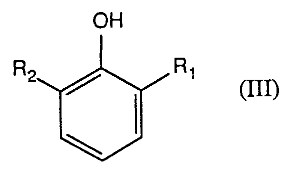 mit einem Nitrit in wässrigem oder wässrig/organischem Medium zu einer Verbindung der Formel II 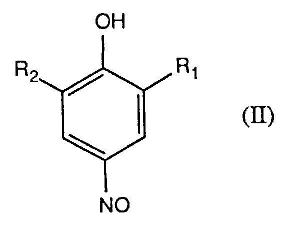 umsetzt, letztere mit einem Lösungsmittel aus der Reaktionslösung extrahiert, sie ohne Isolierung im genannten Lösungmittel in Gegenwart eines Palladium-Katalysators katalytisch zum entsprechenden p-Aminophenol hydriert und letzteres ohne dessen Isolierung mit Cyanurchlorid und einer Verbindung der Formel HSR₃ oder HSR₄ oder einer Mischung von Verbindungen der Formeln HSR₃ und HSR₄ umsetzt.

2. Verfahren nach Anspruch 1, worin man in einem Lösungsmittel arbeitet, das unter den Bedingungen des Hydrierungsschrittes inert ist.

3. Verfahren nach einem der Ansprüche 1 oder 2, worin als Lösungsmittel ein Keton, ein Ester, ein Ether, ein aromatischer oder aliphatischer oder cycloaliphatischer Kohlenwasserstoff, Mischungen solcher Lösungsmittel oder ein Gemisch aus mindestens einem der vorgenannten Lösungsmittel und Wasser verwendet wird.

4. Verfahren nach Anspruch 3, worin als Lösungsmittel Methytisobutylketon, Methylethylketon oder Gemische dieser Lösungsmittel mit Wasser verwendet werden.

5. Verfahren nach einem der Ansprüche 1 bis 4, worin die katalytische Hydrierung bei Temperaturen von 20-100°C, insbesondere 20-80°C, durchgeführt wird.

6. Verfahren nach einem der Ansprüche 1 bis 4, worin die katalytische Hydrierung bei Drucken von 1-200 bar, insbesondere 1-100 bar, durchgeführt wird.

7. Verfahren nach einem der Ansprüche 1 bis 4, worin die Katalysatormenge 0,01 bis 5,0 Gew.-%, insbesondere 0,2 bis 1,5 Gew.-%, bezogen auf die Verbindung der Formel II, beträgt.

8. Verfahren nach einem der Ansprüche 1 bis 4, worin als Hydrierkatalysator Pd auf einem Träger, insbesondere Pd/C, eingesetzt wird.

9. Verfahren nach Anspruch 8, worin der Träger mit 0,5-5 %, insbesondere 0,5-2,0 %, Pd belegt ist.

10. Verfahren nach einem der Ansprüche 1 bis 4, worin R₁ und R₂ unabhängig voneinander Wasserstoff oder C₁-C₁₂-Alkyl bedeuten und R₃ und R₄ unabhängig voneinander für C₁-C₁₂-Alkyl stehen.

11. Verfahren nach einem der Ansprüche 1 bis 4, worin R₄ und R₃ gleich sind.

12. Verfahren nach einem der Ansprüche 1 bis 4, worin R₁ und R₂ gleich sind.

13. Verfahren nach Anspruch 12, worin R₁ und R₂ tert.-Butyl bedeuten.

## Claims

1. A process for the preparation of an alkylhydroxyanilinotriazinethio derivative of formula I 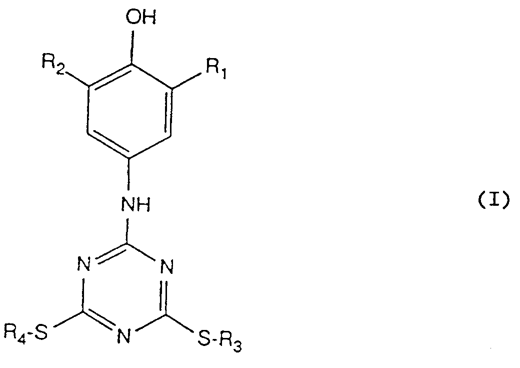 in which R₁ and R₂ are each independently of the other hydrogen, C₁-C₁₂alkyl, C₅-C₆cycloalkyl or phenyl, and
R₃ and R₄ are each independently of the other C₁-C₁₈alkyl, C₅-C₆cycloalkyl, phenyl, phenyl which is substituted by C₁-C₆alkyl and/or hydroxyl, or phenyl-C₁-C₄alkyl, which process comprises reacting a compound of formula III 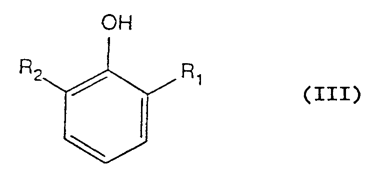 with a nitrite, in aqueous or aqueous/organic medium, to give a compound of formula II 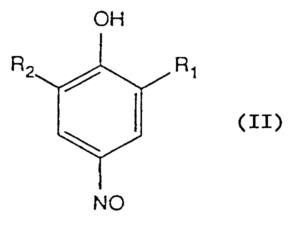 extracting the latter from the reaction solution with a solvent, catalytically hydrogenating it, without isolation, in the cited solvent and in the presence of a palladium catalyst, to give the corresponding p-aminophenol, and reacting the latter, without isolation, with cyanuric chloride and a compound of formula HSR₃ or HSR₄ or a mixture of compounds of formulae HSR₃ and HSR₄.

2. A process according to claim 1, which is carried out in a solvent which is inert under the conditions of the hydrogenation step.

3. A process according to either claim 1 or claim 2, wherein the solvent used is a ketone, an ester, an ether, an aromatic, aliphatic or cycloaliphatic hydrocarbon, a mixture of such solvents or a mixture of at least one of said solvents and water.

4. A process according to claim 3, wherein the solvent used is methyl isobutyl ketone, methyl ethyl ketone or a mixture of said solvents with water.

5. A process according to any one of claims 1 to 4, wherein the catalytic hydrogenation is carried out at temperatures of from 20 to 100°C, especially from 20 to 80°C.

6. A process according to any one of claims 1 to 4, wherein the catalytic hydrogenation is carried out at pressures of from 1 to 200 bar, especially from 1 to 100 bar.

7. A process according to any one of claims 1 to 4, wherein the amount of catalyst is 0.01 to 5.0 % by weight, especially 0.2 to 1.5 % by weight, based on the compound of formula II.

8. A process according to any one of claims 1 to 4, wherein the hydrogenation catalyst used is Pd on a support, especially Pd/C.

9. A process according to claim 8, wherein the amount of Pd on the support is 0.5-5 %, especially 0.5-2.0 %.

10. A process according to any one of claims 1 to 4, wherein R₁ and R₂ are each independently of the other hydrogen or C₁-C₁₂alkyl, and R₃ and R₄ are each independently of the other C₁-C₁₂alkyl.

11. A process according to any one of claims 1 to 4, wherein R₄ and R₃ are identical.

12. A process according to any one of claims 1 to 4, wherein R₁ and R₂ are identical.

13. A process according to claim 12, wherein R₁ and R₂ are tert-butyl.

## Revendications

1. Procédés pour la préparation de dérivés thio d'alkylhydroxyanilino-triazine de formule I 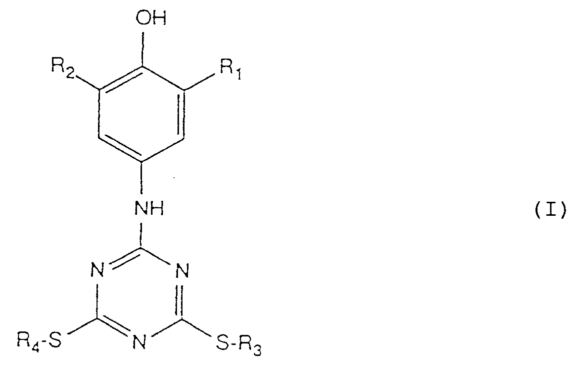 dans laquelle R₁ et R₂, indépendamment l'un de l'autre, signifient l'hydrogène, les alkyle en C₁-C₁₂, cycloalkyle en C₅-C₆ ou phényle, et
R₃ et R₄, indépendamment l'un de l'autre, représentent les alkyle en C₁-C₁₈, cycloalkyle en C₅-C₆, phényle, phényle substitué par des alkyle en C₁-C₆ et/ou hydroxy ou phényl-(alkyle en C₁-C₄), caractérisés en ce qu'on fait réagir un composé de formule III 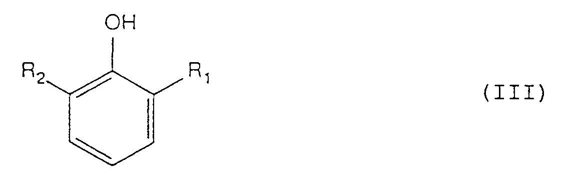 avec un nitrite en milieu aqueux ou aqueux/organique pour obtenir un composé de formule II 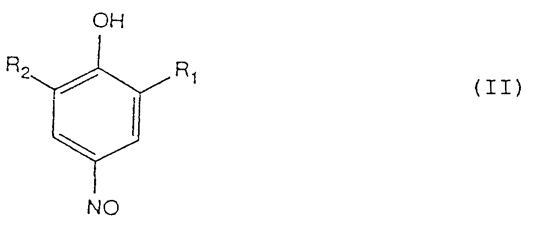 on extrait ce dernier avec un solvant à partir de la solution réactionnelle, on l'hydrogène catalytiquement, sans isolement, dans le solvant cité en présence d'un catalyseur au palladium, en p-aminophénol correspondant et en faisant réagir ce dernier, sans son isolement, avec du chlorure de cyanuryle et un composé de formule HSR₃ ou HSR₄ ou un mélange de composés de formules HSR₃ et HSR₄.

2. Procédé selon la revendication 1, dans lequel on travaille dans un solvant qui est inerte dans les conditions de l'étape d'hydrogénation.

3. Procédé selon une des revendications 1 ou 2, dans lequel on utilise en tant que solvant une cétone, un ester, un éther, un hydrocarbone aromatique ou aliphatique ou cycloaliphatique, des mélanges de tels solvants ou un mélange d'au moins un des solvants précités et d'eau.

4. Procédé selon la revendication 3, dans lequel on utilise en tant que solvants la méthylisobutylcétone, la méthyléthylcétone ou les mélanges de ces solvants avec l'eau.

5. Procédé selon l'une des revendications 1 à 4, dans lequel on met en oeuvre l'hydrogénation catalytique à des températures de 20 à 100 °C, plus particulièrement de 20 à 80 °C.

6. Procédé selon l'une des revendications 1 à 4, dans lequel on met en oeuvre l'hydrogénation catalytique à des pressions de 1 à 200 bars, plus particulièrement de 1 à 100 bars.

7. Procédé selon l'une des revendications 1 à 4, dans lequel la quantité de catalyseur est de 0,01 à 5,0 % en poids, plus particulièrement de 0,2 à 1,5 % en poids par rapport au composé de formule II.

8. Procédé selon une des revendications 1 à 4, dans lequel on utilise en tant que catalyseur d'hydrogénation le Pd sur un support, plus particulièrement le Pd/C.

9. Procédé selon la revendication 8, où le support est revêtu de 0,5 à 5 %, plus particulièrement de 0,5 à 2,0 % de palladium.

10. Procédé selon l'une des revendications 1 à 4, dans lequel R₁ et R₂, indépendamment l'un de l'autre, représentent l'hydrogène ou un alkyle en C₁-C₁₂ et R₃ et R₄, indépendamment l'un de l'autre, représentent un alkyle en C₁-C₁₂.

11. Procédé selon l'une des revendications 1 à 4, dans lequel R₃ et R₄ sont identiques.

12. Procédé selon l'une des revendications 1 à 4, dans lequel R₁ et R₂ sont identiques.

13. Procédé selon la revendication 12, dans lequel R₁ et R₂ représentent le tert-butyle.
